# EUROPÄISCHE PATENTSCHRIFT

(11) **EP 0 529 631 B1**
(45) Veröffentlichungstag und Bekanntmachung des Hinweises auf die Patenterteilung: **14.10.1998**
(21) Anmeldenummer: 92114648.6
(22) Anmeldetag: 27.08.1992
(51) Int. Cl.: C07D 239/68

(54) **Verfahren zur Herstellung von 2-(Methylthio)-dinatriumbarbiturat**
Process for the preparation of 2-(methylthio)-dinatriumbarbiturates
Procédé de préparation de 2-(méthylthio)-dibarbiturates de sodium

(30) Priorität: 30.08.1991 CH 2555/91
(43) Veröffentlichungstag der Anmeldung: 03.03.1993
(73) Patentinhaber: LONZA AG, CH-3945 Gampel/Wallis (CH)
(72) Erfinder: Roduit, Jean-Paul, Dr., CH-Sierre (Kanton Wallis) (CH); Etzensperger, Marcel, CH-Gamsen (Kanton Wallis) (CH); Wellig, Alain, CH-Ried bei Mörel (Kanton Wallis) (CH)
(74) Vertreter: Weinhold, Peter, Dr.

(56) Entgegenhaltungen:
- EP-A- 0 411 276
- EP-A- 0 411 277

## Beschreibung

Die Erfindung betrifft ein neues Verfahren zur Herstellung von 2-(Methylthio)-dinatriumbarbiturat der Formel 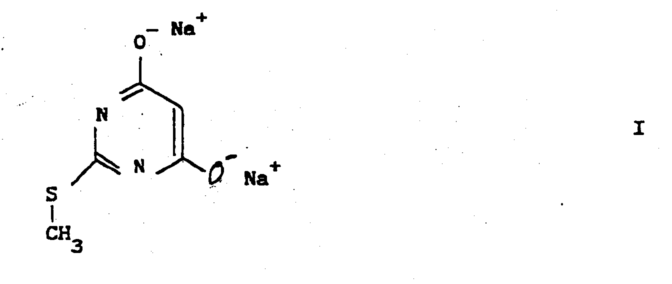 ausgehend von Thioharnstoff, Malonsäuredimethylester und Natriummethanolat.

2-(Methylthio)-dinatriumbarbiturat ist ein wichtiges Zwischenprodukt zur Herstellung von Herbiziden gemäß US-A-4 692 524.

Bekannt ist die Herstellung von Mononatriumthiobarbiturat ausgehend von Malonsäuredimethylester, Thioharnstoff und Natriummethanolat, wobei diese 3 Ausgangsstoffe in einem annähernd molaren Verhältnis von 1:1:1 eingesetzt werden (EP-A-411 277). Desweiteren ist die Folgestufe,die Methylierung dieses Mononatriumthiobarbiturats mit Methylbromid zur 2-(Methylthio)-barbitursäure,bekannt, wobei diese Umsetzung unter erhöhtem Druck durchgeführt wird (EP-A-411 276).

Ein Nachteil dieser 2-stufigen Synthese zur Herstellung von 2-(Methylthio)-barbitursäure besteht darin, dass in der ersten Stufe das Mononatriumthiobarbiturat gebildet wird, wobei dieses dann in der Folgestufe nur unter erhöhtem Druck und erhöhter Temperatur mit Methylbromid zur 2-(Methylthio)-barbitursäure reagiert.

Aufgabe der Erfindung ist es, diese Nachteile zu beseitigen und ein grosstechnisch gangbares Verfahren zur Verfügung zu stellen, bei dem einerseits beide Stufen ohne Druck durchgeführt werden können und 2-(Methylthio)-dinatriumbarbiturat in guter Ausbeute erhalten wird, welches direkt in die Herbizide gemäß US-A-4 692 524 überführt werden kann.

Diese Aufgabe wird mit dem neuen Verfahren gemäss Patentanspruch 1 gelöst.

Erfindungsgemäss wird das Verfahren derart durchgeführt, dass man in der ersten Stufe Thioharnstoff, Malonsäuredimethylester und Natriummethanolat, in Methanol, zum Dinatriumthiobarbiturat umsetzt und dieses in der zweiten Stufe mit Methylbromid, in Gegenwart einer Base, zum Endprodukt gemäss Formel 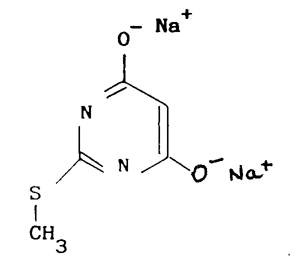 umsetzt.

Die Umsetzung in der ersten Stufe wird mit 2 bis 4 mol Natriummethanolat, vorzugsweise mit 3 mol, bezogen auf 1 mol Thioharnstoff und 1 mol Malonsäuredimethylester, durchgeführt.

Die Umsetzung in der ersten Stufe kann entweder in einem Autoklaven unter einem Druck von 2 bis 4 bar oder unter Normaldruck erfolgen.

Zweckmässig wird die Umsetzung in der ersten Stufe bei einer Temperatur von 65 bis 100°C, vorzugsweise unter Rückfluss, durchgeführt.

Nach einer üblichen Reaktionszeit von 3 bis 5 h kann dann das Dinatriumthiobarbiturat entweder auf fachmännisch übliche Weise isoliert werden oder es kann direkt für die zweite Stufe eingesetzt werden.

Die Umsetzung in der zweiten Stufe wird zweckmässig mit einem leichten Überschuss an Methylbromid durchgeführt. Vorzugsweise werden 1 bis 1,3 mol Methylbromid, bezogen auf 1 mol Dinatriumthiobarbiturat, eingesetzt.

Die Umsetzung in der zweiten Stufe wird in Gegenwart einer Base durchgeführt.
Als Base wird zweckmässig ein Alkalimetallhydroxid eingesetzt. Als Alkalimetallhydroxid wird beispielsweise Natriumhydroxid oder Kaliumhydroxid angewendet.

Die Umsetzung in der zweiten Stufe wird in einem polaren Lösungsmittel, gegebenenfalls in einem polaren Lösungsmittelgemisch, durchgeführt.
Als polare Lösungsmittel können Wasser, niedrig siedende Alkohole, wie beispielsweise Methanol, Ethanol und Isopropanol, oder niedrig siedende Ketone, wie beispielsweise Aceton, angewendet werden.

Als polares Lösungsmittelgemisch kann eine Wasser-Alkohol- bzw. Wasser-Keton-Mischung Verwendung finden ,wie beispielsweise eine Wasser-Isopropanol- oder Wasser-Aceton-Mischung.

Die Reaktionstemperatur liegt in der zweiten Stufe zwischen 0 und 20°C, vorzugsweise zwischen 5 und 15°C.
Die Umsetzung in der zweiten Stufe wird unter Normaldruck durchgeführt.

Nach einer üblichen Umsetzung von 2 bis 3 h in der zweiten Stufe kann dann das Endprodukt gemäss Formel I beispielsweise durch Filtration isoliert werden.

In einer vorzugsweisen Ausführung wird das Verfahren ohne Isolation des Zwischenproduktes (Dinatriumthiobarbiturat) durchgeführt, wobei dann das Endprodukt gemäss Formel I nach einer üblichen Umsetzungsdauer von 2 bis 3 h erhalten wird.

### Beispiel 1:

### a) Herstellung von Dinatriumthiobarbiturat (mit Druck)

In einem Autoklaven (2 l) wurde unter inerter Atmosphäre eine 30%ige methanolische Lösung von Natriummethanolat (432,2 g, 2,4 mol) vorgelegt und mit Methanol (200 g, 6,24 mol) verdünnt.
Anschliessend wurden Thioharnstoff (60,8 g, 0,8 mol) und Malonsäuredimethylester (105,7 g, 0,8 mol) hinzugegeben, und das Ganze wurde unter Rühren auf 100°C erhitzt, wobei sich ein Druck von 3 bar eingestellt hatte.
Nach 3 h wurde die Reaktionsmischung auf 2°C abgekühlt und das Dinatriumthiobarbiturat durch Filtration in Form von weissen Kristallen isoliert.
Es konnten 244 g Feuchtprodukt isoliert werden.
Das Methanol im Filtrat wurde durch Destillation zurückgewonnen.

### b) Herstellung von 2-(Methylthio)-dinatriumbarbiturat

244 g Dinatriumthiobarbiturat wurden in Wasser (550 g) gelöst. Ungelöste Partikel wurden durch Filtration entfernt.
Anschliessend wurde das resultierende Filtrat auf 5°C abgekühlt. Dann wurden eine 40%ige wässrige Natriumhydroxid-Lösung (87,6 g, 0,876 mol) und Isopropanol (65 g, 1,08 mol) hinzugefügt. Hierzu wurde eine zuvor bereitete Lösung von Methylbromid (83,25 g, 0,876 mol) in Isopropanol (249,75 g, 4,155 mol) innerhalb 3 h hinzugegeben.
Anschliessend wurde die Reaktionsmischung für eine weitere Stunde bei 10°C gerührt und innerhalb von 20 min mit Isopropanol (194 g, 3,22 mol) während dem Abkühlen auf 1°C versetzt.
Diese Mischung wurde für weitere 15 min auf 1°C gehalten.
Nach Filtration wurden 249,8 g Feuchtprodukt in Form von weissen Kristallen,entsprechend einer Ausbeute von 90,5%, bezogen auf eingesetztes Dinatriumthiobarbiturat, erhalten.

237,13 g des so erhaltenen 2-(Methylthio)-dinatriumbarbiturats wurden anschliessend in Wasser (239 g) bei 45°C gelöst. Dann wurde innerhalb 10 bis 15 min bei 45°C unter Rühren Isopropanol (237; 3,94 mol) hinzugefügt, wobei das Produkt begann auszukristallisieren. Das so erhaltene weisse kristalline Produkt wurde filtriert und bei 65°C und einem Druck von 0,026 bar getrocknet.
Nach Kristallisation wurden 134,5 g trockenes kristallines Material erthalten, mit einem Gehalt an 2-(Methylthio)-dinatriumbarbiturat von 95,2%, entsprechend einer Ausbeute von 83,5% bezüglich eingesetztem Thioharnstoff.

### Beispiel 2: (Eintopfvariante)

### a) Herstellung von Dinatriumthiobarbiturat (ohne Druck)

72 kg Thioharnstoff (947 mol) wurden in wasserfreiem Methanol (182 l) gelöst. Ausschliessend wurden Malonsäuredimethylester (125 kg; 947 mol) und Natriummethanolat (717 kg; 2840 mol) 21,4% in Methanol zudosiert,und das Ganze wurde unter Rückfluss 3 h lang gerührt. Danach wurde abgekühlt und das Methanol unter Vakuum abdestilliert. Nach Zugabe von destilliertem H₂O (360 l) wurden die restlichen Mengen an Methanol unter Vakuum destilliert.

### b) Herstellung von 2-(Methylthio)-dinatriumbarbiturat

Zu dieser Mischung wurden 30%ige Natronlauge (104,1 l) und Aceton (767 l) hinzugegeben. Anschliessend wurde Methylbromid bei 10°C (98,5 kg; 1036 mol) während 2,5 bis 3 h hinzudosiert. Danach wurde bei 15°C für weitere 2,5 h gerührt. Insgesamt wurden nach Filtration bei 0°C 318,6 kg feuchtes 2-(Methylthio)-dinatriumbarbiurat (97%-ig) erhalten, entsprechend einer Ausbeute von 82,7% bezüglich eingesetztem Thioharnstoff.

## Patentansprüche

1. Verfahren zur Herstellung von 2-(Methylthio)-dinatriumbarbiturat der Formel 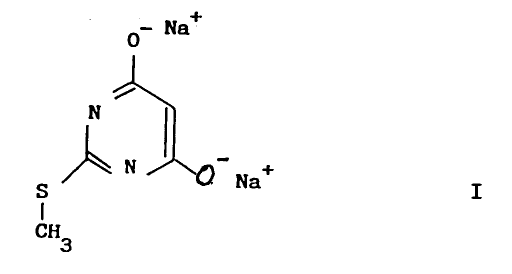 dadurch gekennzeichnet, daß man in der ersten Stufe Thioharnstoff, Malonsäuredimethylester und 2 bis 4 Mol Natriummethanolat, in Methanol zum Dinatriumthiobarbiturat umsetzt und dieses dann in der zweiten Stufe bei Normaldruck und einer Reaktionstemperatur zwischen 0 und 20°C in Gegenwart eines polaren Lösungsmittels oder eines polaren Lösungsmittelgemisches mit Methylbromid und einer Base zum Endprodukt gemäß Formel I umsetzt.

2. Verfahren nach Patentanspruch 1, dadurch gekennzeichnet, daß man die Umsetzung in der ersten Stufe entweder unter Normaldruck oder bei einem Druck von 2 bis 4 bar durchführt.

3. Verfahren nach mindestens einem der Patentansprüche 1 und 2, dadurch gekennzeichnet, daß man die Umsetzung in der ersten Stufe bei Temperaturen von 65 bis 100°C durchführt.

4. Verfahren nach mindestens einem der Patentansprüche 1 bis 3, dadurch gekennzeichnet, daß man in der zweiten Stufe als Base ein Alkalimetallhydroxid einsetzt.

5. Verfahren nach mindestens einem der Patentansprüche 1 bis 4, dadurch gekennzeichnet, daß man die Umsetzung ohne Isolation des Zwischenproduktes durchführt.

## Claims

1. A method for producing 2-(methylthio)-disodiumbarbiturate having the formula 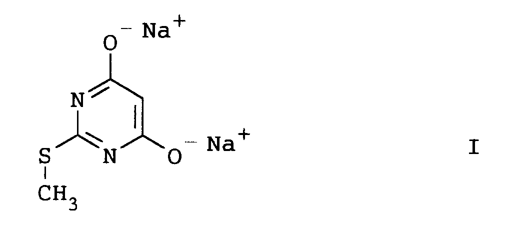 characterised in that in the first stage thiourea, malonic dimethylester and 2 to 4 mol sodium methylate are reacted in methanol to form disodiumthiobarbiturate, with the latter then, in the second stage, being reacted with methylbromide and a base at normal pressure and at a reaction temperature between 0 and 20°C in the presence of a polar solvent or of a polar mixture of solvents, to form the final product in accordance with Formula I.

2. The method according to claim 1, characterised in that reaction in the first stage is performed either under normal pressure or at a pressure from 2 to 4 bar.

3. The method according to at least one of claims 1 and 2, characterised in that reaction in the first stage is performed at temperatures from 65 to 100°C.

4. The method according to at least one of claims 1 to 3, characterised in that in the second stage an alkali metal hydroxide is used as a base.

5. The method according to at least one of claims 1 to 4, characterised in that reaction is carried out without isolation of the intermediate product.

## Revendications

1. Procédé pour la préparation de 2-(méthylthio)-dibarbiturate de sodium de la formule 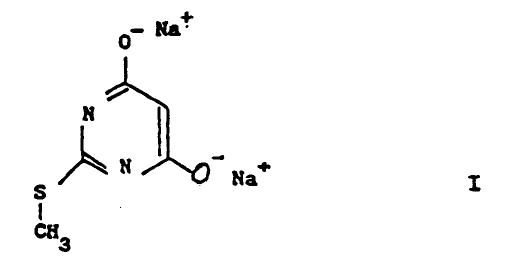 caractérisé en ce que dans une première étape, on transforme la thio-urée, le diméthylester de l'acide malonique et 2 à 4 moles de méthanolat de sodium, dans le méthanol en le dibarbiturate de sodium et dans la deuxième étape, on procède à la réaction de celui-ci à pression normale et à une température de réaction entre 0 et 20°C en présence d'un solvant polaire ou d'un mélange de solvants polaires avec du bromure de méthyle et une base pour obtenir le produit final selon la revendication 1.

2. Procédé selon la revendication 1, caractérisé en ce que dans la première étape, on conduit la réaction à une pression normale ou à une pression de 2 à 4 bars.

3. Procédé selon au moins l'une des revendications 1 et 2, caractérisé en ce que dans la première étape, on conduit la réaction à des températures de 65 à 100°C.

4. Procédé selon au moins l'une des revendications 1 à 3, caractérisé en ce que dans la deuxième étape, on met en oeuvre en tant que base, un hydroxyde des métaux alcalins.

5. Procédé selon au moins l'une des revendications 1 à 4, caractérisé en ce que l'on conduit la réaction sans isoler le produit intermédiaire.
